# EUROPEAN PATENT SPECIFICATION

(11) **EP 2 595 546 B1**
(45) Date of publication and mention of the grant of the patent: **08.04.2015**
(21) Application number: 11749925.1
(22) Date of filing: 22.07.2011
(51) Int. Cl.: A61B 10/02

(54) **ASPIRATION INCISION BIOPSY NEEDLE**
ASPIRATIONSNADEL FÜR INZISIONSBIOPSIE
AIGUILLE DE BIOPSIE POUR ASPIRATION ET INCISION

(30) Priority: 23.07.2010 TR 201006093
(43) Date of publication of application: 29.05.2013
(73) Proprietor: Eren, Orhan, Canakkale (TR)
(72) Inventor: Eren, Orhan, Canakkale (TR); Ozturk, Ali Ozkan, Sariyer, Istanbul (TR)
(74) Representative: Dericioglu, E. Korhan
(86) International application number: PCT/IB2011/053275
(87) International publication number: WO 2012/011079

(56) References cited:
- EP-A2- 1 889 572
- WO-A1-2006/001651
- WO-A1-2009/128796
- WO-A2-2007/110812
- NL-A- 7 607 008

## Description

### Field of the Invention

The present invention relates to an aspiration incision biopsy needle which enables to take samples such as soft tissue specimens and/or cystic fluids, tissue aspirates and blood materials for determining the reasons of anomalous nodules and masses (tumors) in the organs of the human body.

### Background of the Invention

There are two types of biopsies, namely open and closed. Open biopsy is carried out surgically in the concerned organ by opening cutaneous and subcutaneous tissues with a surgical procedure and inspecting visually. However, open biopsy is not preferred much since it requires surgical procedure and it is a highly invasive process. Closed biopsy is a process for taking tissue specimens from target organs by the help of a needle and it is a less invasive process. Closed biopsy is a method which is used more often than open biopsy in the recent years.

There are two types of needle aspiration biopsy, which is a closed biopsy, namely, thick needle aspiration biopsy which is used in bone marrow biopsy and thin needle aspiration biopsy (TNAB) which is performed by a thin needle and is used more often. Thin needle aspiration biopsy is a method which is frequently applied in thyroid and lymphatic glands. In this method, after target lesions in tissues such as thyroid or breast are reached with the needle, with the vacuum generated inside the injection syringe as a result of pulling back the moving part thereof, tissue aspirate containing cells and fluids from the target tissue are first taken into the needle, then to the syringe hollow. In TNAB method, the fact that the needle is made of a single piece causes problems such as failing to take sufficient tissue specimen and obtaining faulty results.

Tru-cut biopsy method is also frequently used as the closed biopsy method. This method is especially preferred in breast and kidney biopsies. Additionally, there are other specific biopsy methods such as Menghini, Abram for liver and pleura biopsies, respectively.

There are some problems encountered in the current tru-cut application method. In this method, application requires experience. With the inner needle biopsy system, the aspiration inner needle with a cavity at the end thereof can be moved towards the target tissue in the case that it is aligned with the end of the outer cylindrical cutter. For this reason, in order to make an incision, first the inner needle and then the outer needle must be maneuvered within the organ. When the needle is inserted into the organ, it should be stopped at a distance before the target tissue. This distance between the said point that is aimed before the target tissue and the target point is as long as the length of the inner needle aspiration reservoir. Aiming this distance requires mastery and it is possible to make errors in reaching the target tissue. When the inner needle is pushed inside, one must aim once more so as to reach the target tissue. Incision process is carried out by pushing the outer needle cylindrical cutting tube and biopsy is taken. After these procedures, the needle is pulled out.

The Japanese patent document no. JP2001000436, an application known in the art, discloses a biopsy needle, which is comprised of an inner needle possessing a cavity at one end and an outer needle, for taking samples from human body. In this needle, the inner needle is tubular and the collected sample is moved within the inner needle with the pressure generated by the injection syringe.

The European patent document no. EP1903945A1, an application known in the art, discloses a biopsy needle comprising an aspiration combination. According to the said document, the aspiration inner tip goes out during the biopsy. It differs from the tru-cut biopsy in that the inner aspiration needle goes out and then is retracted. The said needle is in the style of a modified tru-cut system and samples are collected with this needle by accumulating in the cavity in the inner needle.

The Japanese patent document no. JP2007054449A discloses a method for collecting samples to the tip of the needle by rotating the outer lumen with a rope-like material connected to the needle.

The International patent document no. WO2007110812A2, an application known in the art, discloses a structure which carries out vacuum assisted coaxial incision. Aspiration notch on the inner lumen goes out of the system and carries out vacuuming action, and incision is made with cutting cylinder during its re-entry.

### Summary of the Invention

The objective of the present invention is to provide an aspiration incision biopsy needle that allows performing closed biopsy in all tissues that contain lesions such as organ, mass, LAP (lymphodenopathy); particularly thyroid, breast, lymph node, cystic lesion, and cutaneous/subcutaneous lesions.

Another objective of the present invention is to provide an aspiration incision biopsy needle with a short application time.

A further objective of the present invention is to provide an aspiration incision biopsy needle which may collect sufficient amount of tissue specimen from the target tissue at one time.

Another objective of the present invention is to provide an aspiration incision biopsy needle which enables to perform a plurality of incisions within the tissue at the same session.

Another objective of the present invention is to provide an aspiration incision biopsy needle which enables to use the syringe with one hand.

Another objective of the present invention is to provide an aspiration incision biopsy needle which includes a check valve.

Another objective of the present invention is to provide an aspiration incision biopsy needle which enables to realize whether or not the needle is in the vein.

Another objective of the present invention is to provide an aspiration incision biopsy needle which reduces the risk of bleeding and invasive complication.

### Detailed Description of the Invention

"An Aspiration Incision Biopsy Needle" realized to fulfill the objective of the present invention is illustrated in the accompanying figures wherein,

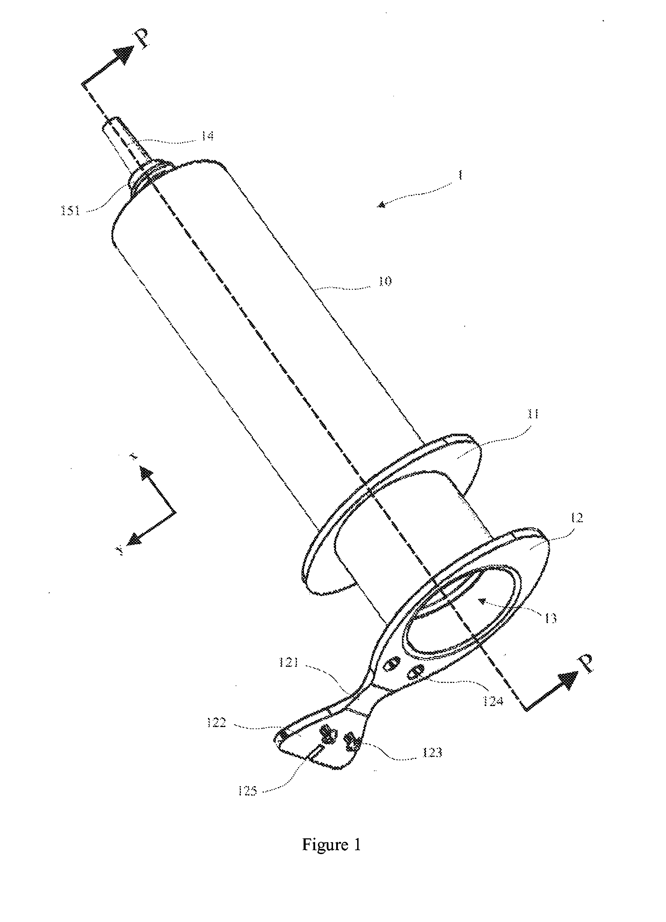

Figure 1 is the perspective view of the injection syringe.

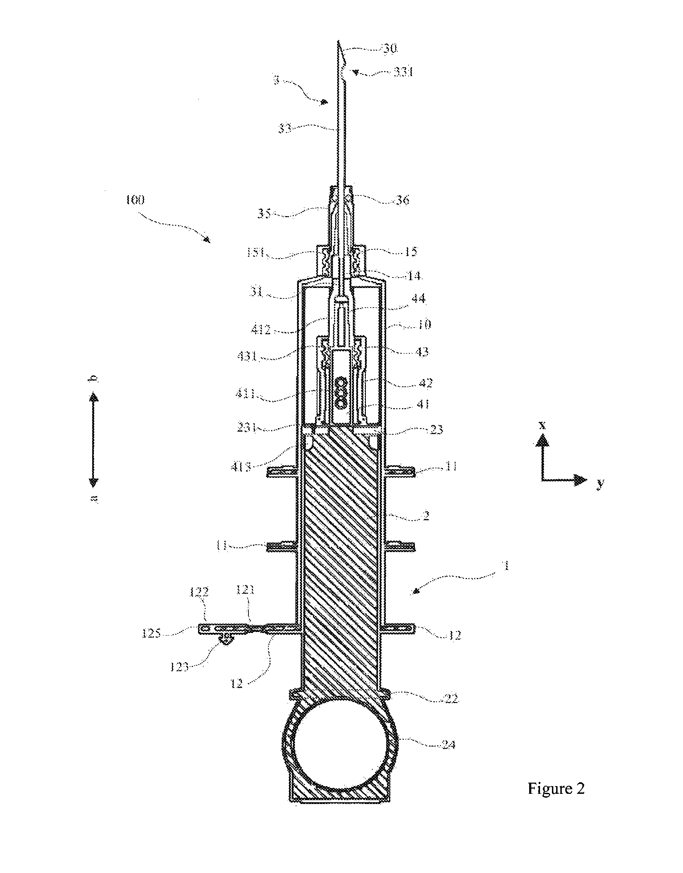

Figure 2 is the view of the P-P section of the biopsy needle shown in Figure 1.

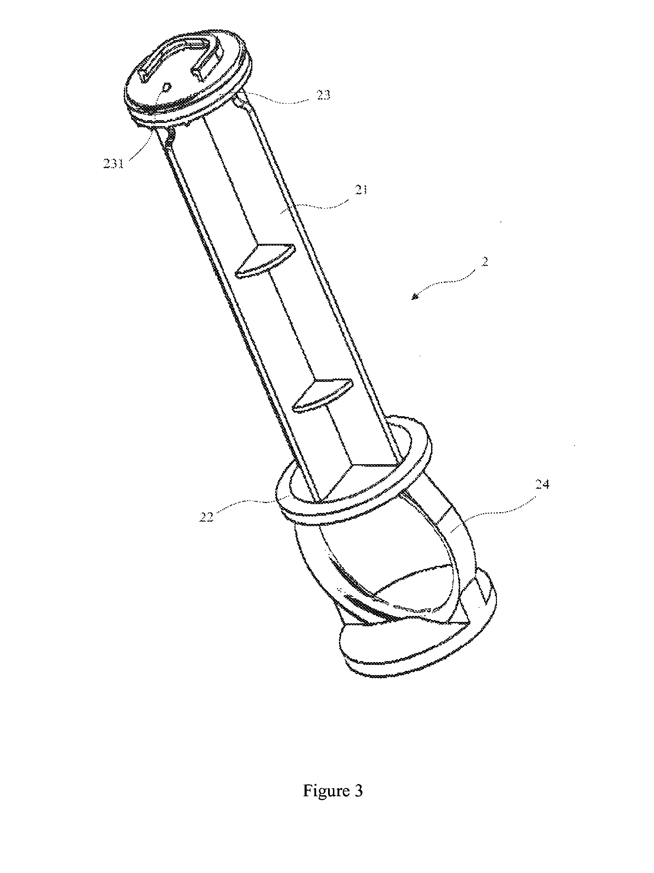

Figure 3 is the perspective view of the moveable part.

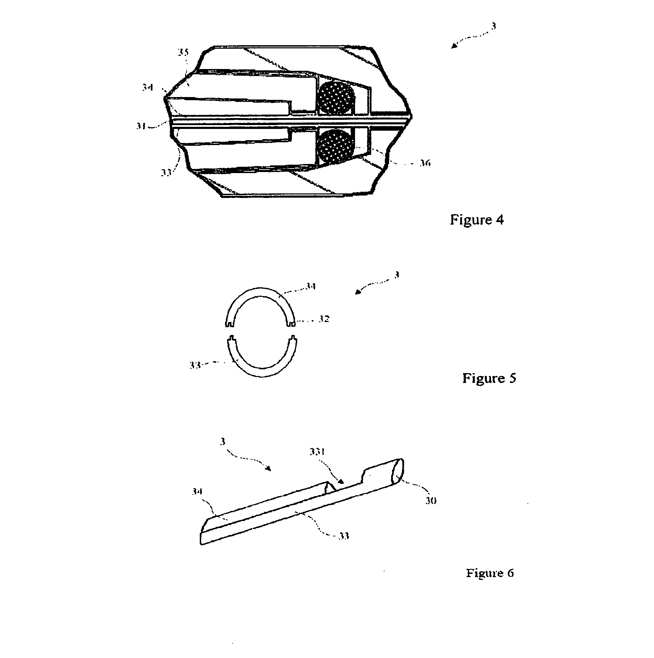

Figure 4 is the view of the P-P section of the needle connection member shown in Figure 1.
Figure 5 is the sectional view of the needle.
Figure 6 is the perspective view of the needle.

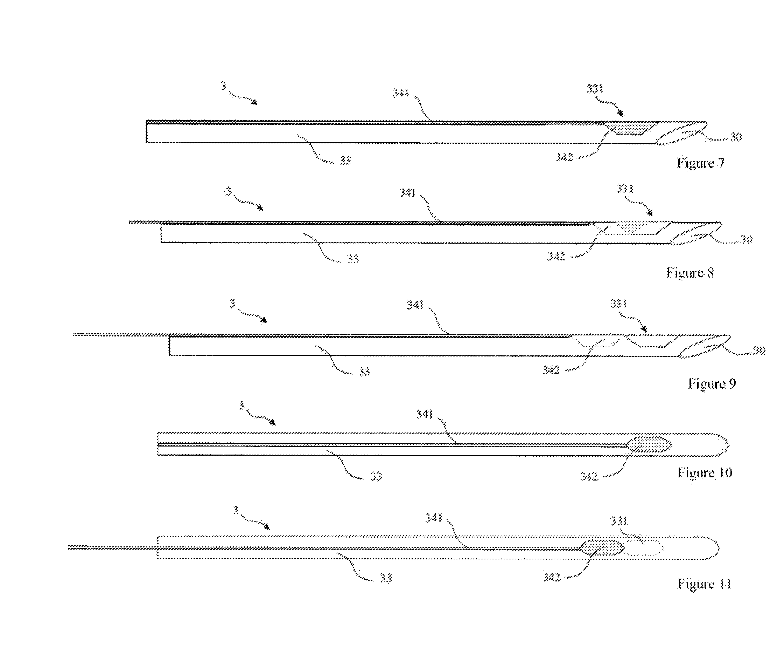

Figure 7 is the side view of the needle when the incision cavity is closed.
Figure 8 is the side view of the needle when the incision cavity is half-closed.
Figure 9 is the side view of the needle when the incision cavity is open.
Figure 10 is the top view of the needle when the incision cavity is closed.
Figure 11 is the top view of the needle when the incision cavity is open.

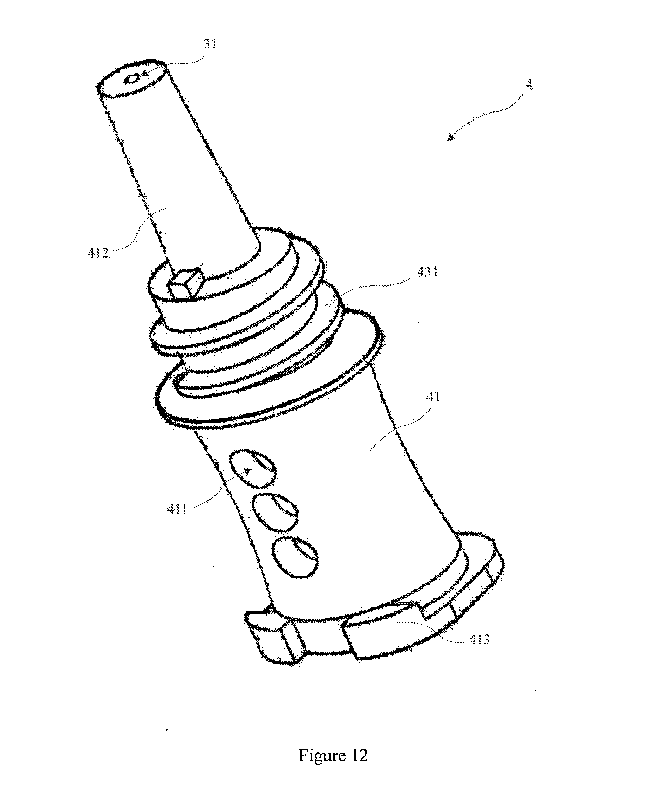

Figure 12 is the perspective view of the check valve shaft.

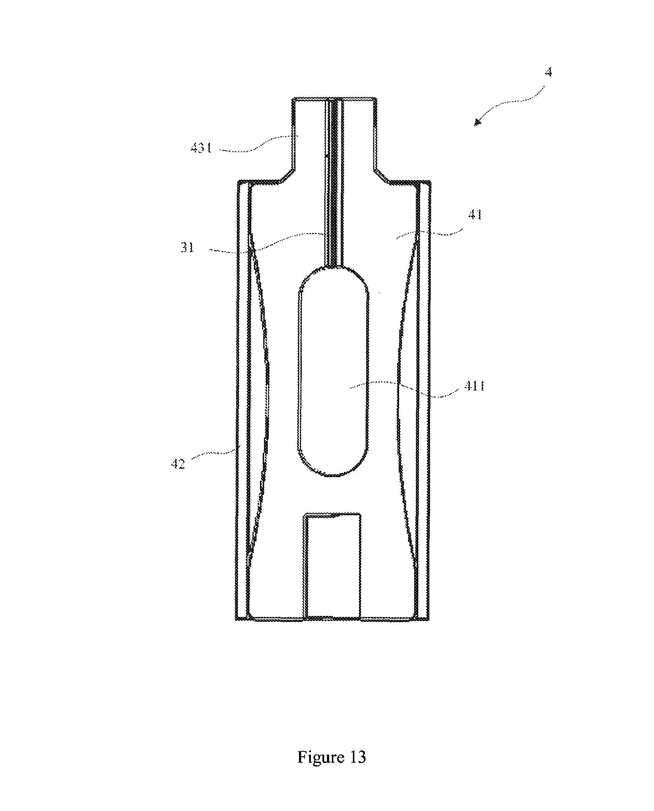

Figure 13 is the view of the P-P section of the check valve shown in Figure 1.

The components shown in the figures are each given reference numerals as follows:
100. Aspiration incision biopsy needle
1. Injection syringe
10. Injection syringe body
11. Holder
12. Stopper
   121. Foldable form
   122. Lug
   123. Tab
   124. Slot
   125. Recess
13. Hollow
14. Injection syringe nozzle
15. Fixing member
   151. Connection hole
2. Moveable part
21. Vane
22. Pressure surface
23. Sealing member
   231. Discharge channel
24. Moveable part handle
3. Needle
30. Needle head
31. Transfer line
32. Movement line
33. Lower part
   331. Incision cavity
34. Upper part
   341. Plate
   342. Cutting edge
35. Connection member
36. Seal
4. Check valve
41. Shaft
   411. Reservoir
   412. Shaft head
   413. Protrusion
42. Tube
43. Fixing member
   431. Connection hole
44. Connection member

The biopsy needle assembly (100) of the present invention essentially comprises
- at least one hollow injection syringe (1),
- at least one moveable part (2) which enables to perform incision by moving within the injection syringe (1),
- at least one needle (3), at least one part of which is connected to the injection syringe (1), and which enters into the body and enables to take samples, and
- at least one check valve (4), which is fixed on at least one part of the needle (3), is mounted to the moveable part (2), and in which the samples that are taken are collected.

The injection syringe (1) comprises at least one hollow injection syringe body (1),
- at least one holder (11) which is located on the lower part of the injection syringe body (10),
- at least one stopper (12) which is located on the injection syringe body (10),
- at least one foldable form (121) provided on at least one side of the stopper (12),
- at least one lug (122) connected to the foldable form (121),
- at least one tab (123) which is located on the surface of the lug (122),
- at least one slot (124) which is located on the surface of the stopper (12),
- at least one recess (125) which is located on the end part of the lug (125),
- at least one hollow (13) within the injection syringe body (10),
- at least one injection syringe nozzle (14) which is located on the upper part of the injection syringe body (10).

The needle assembly (100) of the present invention also comprises
- at least one fixing member (15) mounted on the injection syringe nozzle (14),
- a connection hole (151) located on the injection syringe nozzle (14).

The moveable part (2) comprises at least two vanes (21) thereon,
- at least one pressure surface (22) on the moveable part (2),
- at least one sealing member (23) which is located on one end of the moveable part (2),
- at least one discharge channel (231) located on the sealing member (23),
- at least one moveable part holder (24) located on the pressure surface (22).

The needle (3) comprises a needle head (30) on its end that enters into the body,
- at least one lower part (33) comprised by the needle (3),
- at least one upper part (33) which is comprised by the needle (3), and moves on the lower part (33),
- at least one transfer line (33) which is located in between the lower part (33) and the upper part (34) in the needle (3),
- at least one movement line (32) located on the upper part (34),
- at least one incision cavity (331) located on the lower part (33),
- at least one plate (341) connected to the injection syringe nozzle (14),
- at least one cutting edge (342) located on the end of the plate (341),
- at least one connection member (35) located between the needle (3) and the injection syringe nozzle (14),
- at least one seal (36) located in the connection member (35).

The check valve (4) comprises at least one shaft (41) which is located therein,
- at least one sample collection reservoir (411) located on the shaft (41),
- at least one shaft head (412) which is located on the end of the shaft (41) and which is like an extension extending from the shaft (41),
- at least one protrusion (413) which is located on the lower part of the shaft (41),
- at least one tube (42) which covers the shaft (41),
- at least one fixing member (43) mounted on the shaft head (412),
- a connection hole (431) located on the shaft head (412),
- at least one connection member (44) located between the injection syringe nozzle (14) and the shaft head (412).

In the biopsy needle (100) of the present invention, the incision cavity (331) provided on the needle (3) is inserted until the tissue, from which samples will be taken.

In the following parts of the description, the movement direction of the moveable part (2) in the direction where it covers the hollow (13) will be referred to as direction a, and the movement direction in the direction where it forms vacuum be referred to as direction b.

As a result of moving the moveable part (2) in directions a and b, thus moving the upper part (34) of the needle (3) in directions a and b, samples are taken from the tissue that remains between the lower part (33) and the upper part (34).

In the biopsy needle (100) of the present invention, the moveable part (2) can move within the hollow (13) that extends in directions a and b. In the preferred embodiment of the invention, a piston is used as the moveable part (2). In another embodiment of the invention, a motor is used as the moveable part (2) in order to be able to take samples in series.

Upon exerting pressure on the holder (11) located on the injection syringe (1) and on the pressure surface (22) located on the moveable part (2), the moveable part (2) moves in direction a. The moveable part (2) can be moved in direction b, by holding the moveable part handle (24) located on the moveable part (2). In the preferred embodiment of the invention, there are two holders (11) provided on the injection syringe (1). This way, the biopsy needle (100) can be used with one hand.

In the biopsy needle (100) of the present invention, the check valve (4) is fastened to the moveable part (2) by means of the protrusions (413) below the check valve (4) shaft (41). The upper part (34) of the needle (39) is fastened to the check valve (4) through the transfer line (31) hole on the shaft head (412). By means of this form of connection, movements of the moveable part (2) in directions a and b are directly conveyed to the upper part (34) of the needle (3). Rotation of the moveable part (2) and the upper part (34), which is fastened to the moveable part (2), about x axis may lead to failure to close the incision cavity (331) completely.

There is provided a stopper (12) which prevents the moveable part (2) from rotating in x axis and is located on the injection syringe (1). The stopper (12) comprises at least one foldable form (121) that ensures folding, at least one lug (122) fastened to the foldable form (121), at least one tab (123) on the lug (122) that enables to hold on, at least one slot (124) on the stopper (12) that the tab (123) may fit into, and at least one recess (125) that enables the lug (122) to hold on to the vane (21). The lug (122), which comes onto the stopper (12) by means of the foldable form (121), holds the vane (21) by means of the recess (125) thereof and prevents the moveable part (2) from rotating in x axis.

During the movements of the moveable part (2) and the upper part (34) in directions a and b, by means of the movement line (32) on the upper part (34), the upper part (34) can move on the lower part (33) along the x axis. In the preferred embodiment of the invention, the upper part (34) is a uniform part and is in the form of a cut hollow semicylinder and the part thereof that coincides with the incision cavity (331) has a cutting edge (342). In another embodiment of the invention, the upper part (34) is comprised of a pusher plate (341) until the incision cavity (331), and a cutting edge (342) at the end of the said plate (341) so as to cover the incision cavity (331).

The needle (3) is connected to the injection syringe (1) from the injection syringe nozzle (14) by means of a connection member (35) holding the needle (3). The said connection member (35) is fixed to the injection syringe (1) from the connection hole (151) at the injection syringe nozzle (14) by means of a fixing member (35). In the connection member (35), there is provided a seal (36) that surrounds the needle (3) for preventing leakages that might occur between the lower (33) and upper parts (34) of the needle (3).

By means of the transfer line (31) that passes through the upper part (34), the samples that are taken arrive at the check valve (4), and here, upon passing through the shaft head (412), they are collected in the reservoir (411) located on the shaft (41). There is provided a tube (42) around the shaft (41) for preventing the said sample that is collected in the reservoir (411) from going into the hollow (13). There is a connection member (44) in the hollow (13) that provides the connection between the shaft head (412) and the injection syringe nozzle (14). The said connection member (44) is connected to the shaft (41) from the connection hole (431) on the shaft (41) by means of a fixing member (43).

When the biopsy needle (100) of the present invention is being used, firstly the moveable part (2) is moved in direction a, and when the incision cavity (331) is in closed position, the needle (3) is moved forward until the incision cavity (331) enters into the tissue from which samples will be taken. The moveable part is moved in direction b by being held from its handle (24) and getting support from the holder (11) on the injection syringe body (10). The upper part (2), which moves in direction b with the movement of the moveable part (2), opens the incision cavity (331). Tissue enters into the incision cavity (331) due to the pressure of the tissues in the vicinity and the vacuum provided by the moveable part (2). When the moveable part (2) and the upper part (34) are moved again in direction a, by means of the cutting edge (342) of the upper part (34), the skin is cut along the incision cavity (331) and taken to the transfer line (31). The moveable part (2) is then pulled again in direction a and thereby vacuum is formed, and the collected tissue specimen and fluid sample move along the transfer line (31) and arrive at the check valve (4). In the meantime, by means of the fact that vacuum is formed again and the incision cavity (331) is opened, another new sample can be taken. The high pressure, which occurs in the hollow (13) as a result of repeating the procedure of taking samples, is eliminated by means of the discharge channel (231) located on the sealing member (23). The samples which move along the transfer line (31) and arrive at the check valve (4) pass through the shaft head (412) and are collected in the reservoir (411) located on the shaft (41). At the end of the procedure, the moveable part (2) and the upper part (34) are again moved in direction a and thereby the incision cavity (331) is covered and this way it is taken out of the skin. Firstly the fixing member (15) of the needle (3) is loosened and the needle (3) is removed. The moveable part (62), the check valve (4) connected to the moveable part (2) by protrusions (413), and the upper part (34) connected to the check valve (4) via a fixing member (43) are removed from the hollow (13). Firstly the upper part (34) fixing member (43) is loosened and removed and thereby the check valve (4) is separated from the moveable part (2). Finally, the samples in the check valve (4) reservoir (411) are taken out by separating the pieces of the check valve (4).

Within the scope of these basic concepts, it is possible to develop various embodiments of the inventive biopsy needle (100). The invention can not be limited to the examples described herein; it is essentially as defined in the claims.

## Claims

1. A biopsy needle (100), which enables to take samples such as soft tissue specimens and/or cystic fluids, tissue aspirates and blood for determining the reasons of anomalous nodules and masses (tumors) in the organs of the human body, comprising
- at least one hollow injection syringe (1),
- at least one moveable part (2) which enables to perform incision by moving within the injection syringe (1),
- at least one needle (3), at least one part of which is connected to the injection syringe (1), and which enters into the body and enables to take samples, and **characterized by**
- at least one check valve (4), which is fixed on at least one part of the needle (3), is mounted to the moveable part (2), and in which the samples that are taken are transferred unidirectional.

2. A biopsy needle (100) according to Claim 1, **characterized by** a needle (3) comprising at least one needle head (30) which enables to enter into the body, at least one transfer line (31) that is followed by the sample that is taken, at least one movement line (32) in which the movement is performed when carrying out the incision, at least one lower part (33) which surrounds the transfer line (31), at least one upper part (34) in or on the lower part (33) moving in direction a and b along the movement line (32), at least one connection member (35) which enables connection to the injection syringe (1), a seal (36) which is located in the connection member (35) and prevents the leakages that might occur between the lower part and upper part.

3. A biopsy needle (100) according to any one of the preceding claims, **characterized by** a needle (3) comprising at least one incision cavity (331) which is provided on the lower part (33) and which is the size of the tissue specimen that will be incised.

4. A biopsy needle (100) according to any one of the preceding claims, **characterized by** the needle (3) comprising an upper part (34) having at least one plate (341); which is fastened to the moveable part (2) and conveys the movement it receives from the moveable part (2); and a cutting edge (342) which is located at the end of the plate (341) and is used for incising.

5. A biopsy needle (100) according to Claims 1 to 3, **characterized by** the needle (3) comprising an upper part (34) in the form of a cut cylinder which has a cutting edge (342) for incising.

6. A biopsy needle (100) according to any of the preceding claims, **characterized by** an injection syringe (1) comprising at least one injection syringe body (10) that forms the main structure, at least one holder (11) which provides support to the movement of the moveable part (2), at least one stopper (12) which enables the moveable part (2) to move in a fixed axis, at least one hollow (13) in which the moveable part (2) moves, at least one injection syringe nozzle (14) to which the lower part (33) is connected, at least one fixing member (15) that enables to fix the lower part (33).

7. A biopsy needle (100) according to Claim 6 **characterized by** an injection syringe (1) comprising at least two holders (11) to provide the ease of ability to use with one hand.

8. A biopsy needle (100) according to Claim 6 **characterized by** a stopper (12) comprising at least one foldable form (121) connected to a holder (11), at least one lug (122) connected to the foldable form (121), at least one tab (123) which is provided on the lug (122) and is used for fixing, at least one slot (124) which is provided on the holder (11) and holds the tab (123), at least one recess (125) which is provided on the lug (122) and holds the moveable part (2) to prevent rotation of the moveable part (2).

9. A biopsy needle (100) according to any one of the preceding claims, **characterized by** the injection syringe (1) comprising at least one connection hole (151) which is located on the injection syringe nozzle (14) and holds the fixing member (15).

10. A biopsy needle (100) according to any one of the preceding claims, **characterized by** the moveable part (2) comprising at least two vanes to prevent skidding in the hollow (13), at least one sealing member (23) which prevents escape of the air to form vacuum; and at least one pressure surface (22) that provides support for the movement of the moveable part (2) in the direction where it covers the hollow (13) and at least one moveable part handle (24) which provides support for the movement of the moveable part (2) in the direction where it forms vacuum.

11. A biopsy needle (100) according to Claims 1 to 9 **characterized by** the moveable part (2) wherein a motor is used for taking samples in series.

12. A biopsy needle (100) according to any one of the preceding claims, **characterized by** a moveable part (2) which comprises at least one discharge channel (231) on the sealing member (23) for discharging the high pressure that might form in the hollow (13).

13. A biopsy needle (100) according to any one of the preceding claims, **characterized by** a check valve (4) comprising at least one shaft (41) connected to the end of the moveable part (2), at least one tube (42) which surrounds the shaft (41) and prevents sample loss, at least one fixing member (43) which fixes the upper part (34) to the shaft (41), at least one connection member (44) which provides the connection between the injection syringe nozzle (14) and the shaft (41).

14. A biopsy needle (100) according to any one of the preceding claims, **characterized by** a check valve (4) comprising a shaft (41) that has at least one reservoir (411) which is located on the shaft (41) and in which the samples that are taken are collected; at least one shaft head (412) which extends from the end of the shaft (41) and through which a transfer line (33) passes; at least one protrusion (413) which is located on the lower part of the shaft (41) and enables the shaft (41) to hold on to the moveable part (2).

15. A biopsy needle (100) according to any one of the preceding claims, **characterized by** a check valve (4) comprising at least one connection hole (431) which is located on the shaft head and holds the fixing member (43).

## Patentansprüche

1. Eine Biopsie-Nadel (100), welche die Entnahme von Proben wie Einzelproben und/oder zystische Flüssigkeiten, Gewebeaspirata und Blut zur Bestimmung der Ursachen von anomalen Knölchen und Massen (Tumore) in den Organen des menschlichen Körpers ermöglicht, umfassend
- mindestens eine hohle Injektionsspritze (1),
- mindestens einen beweglichen Teil (2), der die Inzision durch Bewegung innerhalb der Injektionsspritze (1) ermöglicht,
- mindestens eine Nadel (3), deren zumindest ein Teil an der Injektionsspritze (1) angeschlossen ist und welches in den Körper eindringt und ermöglicht Proben zu entnehmen und **gekennzeichnet durch**
- mindestens ein Rückschlagventil (4), welches auf mindestens einem Teil der Nadel (3) befestigt ist und worin die entnommenen Proben in einer Richtung verteilt sind.

2. Eine Biopsie-Nadel (100) nach Anspruch 1, **gekennzeichnet durch** eine Nadel (3) umfassend mindestens einen Nadelkopf (30), der das Eindringen in den Körper ermöglicht, mindestens eine Überweisungsleitung (31), die **durch** die entnommene Probe verfolgt wird, mindestens eine Bewegungsleitung (32), in der die Bewegung erfolgt, wenn die Inzision verwirklicht wird, mindestens ein unteres Teil (33), das die Überweisungsleitung (31) umgibt, mindestens ein oberes Teil (34) in bzw. auf dem unteren Teil (33, das sich in Richtung a und b entlang der Bewegungsleitung (32) bewegt, mindestens ein Befestigungsglied (35), das die Verbindung zur Injektionsspritze (1) ermöglicht, eine Dichtung (36), welche im Befestigungsglied (35) angeordnet ist und das Auslaufen verhindert, das zwischen dem unteren Teil und dem oberen Teil entstehen konnte.

3. Eine Biopsie-Nadel (100) nach einem der vorangehenden Ansprüche, **gekennzeichnet durch** eine Nadel (3), die zumindest eine Inzisions-Aussparung (331) aufweist, welche auf dem unteren Teil (33) vorgesehen ist und welche das Ausmaß von Gewebeprobe hat, die eingeschnitten werden sollte.

4. Eine Biopsie-Nadel (100) nach einem der vorangehenden Ansprüche, **gekennzeichnet durch** eine Nadel (3) umfassend ein unteres Teil (34), das mindestens eine Platte (341) aufweist, welche am beweglichen Teil (2) befestigt ist und die Bewegung überträgt, die sie von dem beweglichen Teil (2) erhält; und eine Schnittkante (342), die am Ende der Platte (341) angeordnet ist und zur Inzision angewendet wird.

5. Eine Biopsie-Nadel (100) nach Ansprüche 1 bis 3, **gekennzeichnet durch** die Nadel (3) umfassend ein oberes Teil (34) in Form einer Walzensäge, welche zur Inzision eine Schnittkante (342) aufweist.

6. Eine Biopsie-Nadel (100) nach einem der vorangehenden Ansprüche, **gekennzeichnet durch** eine Injektionsspritze (1) umfassend zumindest einen Injektionsspritzenkörper (10), welcher die Hauptstruktur bildet, mindestens einen Griff (11), der eine Unterstützung für die Bewegung des beweglichen Teils (2) ermöglicht, mindestens einen Anschlag (12), der dafür sorgt, dass das bewegliche Teil (2) sich auf einer festen Achse bewegt, mindestens eine Aussparung (13), in der das bewegliche Teil (2) sich bewegt, mindestens einen Injektionsspritze-Stutzen (14), an dem das untere Teil (33) angeschlossen ist, mindestens ein Befestigungsglied (15), das die Befestigung des unteren Teils (33) ermöglicht.

7. Eine Biopsie-Nadel (100) nach Anspruch 6, **gekennzeichnet durch** eine Injektionsspritze (1) umfassend mindestens zwei Griffe (11), um die Bequemlichkeit der Bedienung mit einer Hand zu ermöglichen.

8. Eine Biopsie-Nadel (100) nach Anspruch 6, **gekennzeichnet durch** einen Anschlag (12) aufweisend mindestens eine zusammenklappbare Form (121), die an einem Griff (11) angeschlossen ist, mindestens einen Bügel (122), der mit der zusammenklappbaren Form (121) verbunden ist, mindestens einen Tab (123) welcher auf dem Bügel (122) vorgesehen ist und zur Befestigung eingesetzt wird, mindestens einen Einschnitt (124), der auf dem Griff (11) vorgesehen ist und den Tab (123) festhält, mindestens eine Nische (125), welche auf dem Bügel vorgesehen ist und das bewegliche Teil (2) festhält, um die Verdrehung des beweglichen Teils (2) zu verhindern.

9. Eine Biopsie-Nadel (100) nach einem der vorangehenden Ansprüche, **gekennzeichnet durch** die Injektionsspritze (1) aufweisend mindestens eine Verbindungsöffnung (151), welche auf dem Stutzen der Injektionsspritze (14) angeordnet ist und das Befestigungsglied (15) festhält.

10. Eine Biopsie-Nadel (100) nach einem der vorangehenden Ansprüche, **gekennzeichnet durch** das bewegliche Teil (2) umfassend mindestens zwei Flügeln, um das Rutschen in der Aussparung (13) zu verhindern, mindestens ein Gleitstück (23), welches verhindert, dass die Luft ausströmt und somit sich ein Vakuum ausbildet; und mindestens eine Druckfläche (22), die für die Bewegung des beweglichen Teils (2) für eine Unterstützung sorgt, und zwar in der Richtung, wo es die Aussparung (13) abdeckt und mindestens einen Griff (24) für bewegliches Teil, der für die Bewegung des beweglichen Teils (2) eine Unterstützung ermöglicht, und zwar in der Richtung wo es ein Vakuum bildet.

11. Biopsie-Nadel (100) nach Ansprüche 1 bis 9 **gekennzeichnet durch** das bewegliche Teil (2), wobei zur serienmäßigen Probenentnahme ein Motor eingesetzt wird.

12. Eine Biopsie-Nadel (100) nach einem der vorangehenden Ansprüche, **gekennzeichnet durch** ein bewegliches Teil (2), welches mindestens einen Ablaufkanal (231) auf dem Dichtungselement (23) zum Entladen des Hochdrucks aufweist, welcher sich in der Aussparung bilden könnte.

13. Eine Biopsie-Nadel (100) nach einem der vorangehenden Ansprüche, **gekennzeichnet durch** ein Verbindungsglied (44), welches die Verbindung zwischen dem Stutzen (14) der Injektionsspritze und dem Schaft (41) ermöglicht.

14. Eine Biopsie-Nadel (100) nach einem der vorangehenden Ansprüche, **gekennzeichnet durch** ein Rückschlagventil (4), das einen Schaft (41) mit mindestens einem Behälter (411) aufweist, welcher auf dem Schaft (41) angeordnet ist und in welchem die entnommenen Proben gesammelt sind; mindestens einen Schaftkopf (412), welcher sich vom Ende des Schafts (41) erstreckt und **durch** den eine Überweisungsleitung (33) hindurch geführt ist; mindestens einen Vorsprung (413), der im unteren Bereich des Schafts (41) angeordnet ist und es ermöglicht, dass der Schaft (41) sich auf dem beweglichen Teil (2) hält.

15. Eine Biopsie-Nadel (100) nach einem der vorangehenden Ansprüche, **gekennzeichnet durch** ein Rückschlagventil (4) aufweisend mindestens eine Verbindungsöffnung (431), welche auf dem Schaftkopf angeordnet ist und das Befestigungsglied (43) festhält.

## Revendications

1. Une aiguille de biopsie (100) permettant à échantillonner tels que les échantillons de tissu mou et/ou des fluides cystiques, des fluides d'aspiration de la tissue, et de sang afin de déterminer les raison des nodules et masse anormales (les tumeurs) dans les organes du corps humain, comportant:
- au moins une seringue creuse à injection (1),
- au moins une pièce mobile (2) permettant à réaliser une incision en se déplaçant à l'intérieur de la seringue à injection (1),
- au moins une aiguille (3) dont au moins une partie est liée à la seringue à injecter et qui entre dans le corps et permet à échantillonner, et **caractérisée par**:
- au moins un clapet anti-retour (4) fixé sur au moins une partie de l'aiguille (4), monté à la pièce mobile, et dans lequel les échantillons sont transférés dans une façon unidirectionnelle.

2. Une aiguille de biopsie (100) selon la revendication 1, **caractérisée par** une aiguille (3) comportant au moins une tête d'aiguille (30) permettant à l'entrée à l'intérieur du corps, au moins une ligne de transfert (31) suivie d'une prise d'échantillon, au moins une ligne de déplacement (32) à l'intérieur de laquelle on se déplace pendant qu'on effectue l'incision, au moins une partie inférieure (33) entourant la ligne de transfert (31), au moins une partie supérieure (34) dans ou sur la partie inférieure (33) se déplaçant dans la direction (a et b) le long de la ligne de déplacement (32), au moins un élément de raccordement (35) permettant à relier la seringue à injection (1), un joint (36) placé à l'intérieur de l'élément de raccordement (35) et empêchant les fuites qui pourraient survenir entre la partie inférieure et la partie supérieure.

3. Une aiguille de biopsie (100) selon l'une quelconque des revendications précédentes, **caractérisée par** une aiguille (3) comportant au moins une cavité d'incision (331) fournie sur la partie inférieure (33) et qui est la dimension de l'échantillon de tissu à inciser.

4. Une aiguille de biopsie (100) selon l'une quelconque des revendications précédentes, **caractérisée par** l'aiguille (3) comportant une partie supérieure (34) possédant au moins une plaque (341) fixée à la pièce mobile (2) et transférant le mouvement qu'elle a reçue de la pièce mobile (2), et un côté tranchant (342) se trouvant à l'extrémité de la plaque (341) et utilisé pour l'incision.

5. Une aiguille de biopsie (100) selon les revendications 1 à 3, **caractérisée par** l'aiguille (3) comportant une partie supérieure (34) sous la forme d'un cylindre à couper ayant un côté tranchant (342) pour l'incision.

6. Une aiguille de biopsie (100) selon l'une quelconque des revendications précédentes, **caractérisée par** un seringue à injection (1) comportant au moins un corpos de seringue à injection (10) formant la structure principale, au moins un support (11) supportant le mouvement de la pièce mobile (2), au moins un bouchon (12) permettant à la pièce mobile (2) de se déplacer dans un axe fixe, au moins un creux (13) dans lequel se déplace la pièce mobile (2), au moins un injecteur (14) de seringue à injection auquel on relie la partie inférieure (33), au moins un élément de raccordement (15) permettant au raccordement de la partie inférieure (33).

7. Une aiguille de biopsie (100) selon la revendication 6, **caractérisée par** une seringue à injection (1) comportant au moins deux supports (11) pour faciliter la capacité d'utilisation par une seule main.

8. Une aiguille de biopsie (100) selon la revendication 6, **caractérisée par** un bouchon (12) comportant au moins une forme repliable (121) reliée à un support (11), au moins un ergot (122) relié à la forme repliable (121), au moins un onglet (123) fourni sur l'ergot (122) et utilisé pour la fixation, au moins un rainure (124) fournie sur le support (11) et tenant l'onglet (123), au moins un logement (125) fourni sur l'ergot (122) et tenant la pièce mobile (2) pour empêcher la rotation de la pièce mobile (2).

9. Une aiguille de biopsie (100) selon l'une quelconque des revendications précédentes, **caractérisée par** un seringue à injection (1) comportant au moins un trou de raccordement (151) se trouvant sur l'injecteur (14) de seringue à injection et tenant l'élément de raccordement (15).

10. Une aiguille de biopsie (100) selon l'une quelconque des revendications précédentes, **caractérisée par** une pièce mobile (2) comportant au moins deux soupapes pour empêcher le glissement dans le creux (13), au moins un élément de joint d'étanchéité (23) empêchant la fuite de l'air pour former le vide, et au moins un surface de pression (22) supportant le mouvement de la pièce mobile (2) dans la direction où elle couvre le creux (13), et au moins une poignée (24) de pièce mobile fournissant le support pour le mouvement de la pièce mobile (2) dans la direction où elle crée le vide.

11. Une aiguille de biopsie (100) selon les revendications 1 à 9, **caractérisée par** la pièce mobile (2) dans laquelle est utilisé un moteur pour échantillonner en séries.

12. Une aiguille de biopsie (100) selon l'une quelconque des revendications précédentes, **caractérisée par** une pièce mobile (2) comportant au moins un canal de décharge (231) sur l'élément de joint d'étanchéité (23) pour décharger l'haute pression qui pourrait apparaitre dans le creux (13).

13. Une aiguille de biopsie (100) selon l'une quelconque des revendications précédentes, **caractérisée par** un clapet anti-retour (4) comportant au moins un arbre (41) relié à l'extrémité de la pièce mobile (2), au moins un tube (42) entourant l'arbre (41) et empêchant la perte d'échantillon, au moins un élément de fixation (43) fixant la partie supérieure (34) à l'arbre (41), au moins un élément de raccordement (44) fournissant la connexion entre l'injecteur (14) de seringue à injection et l'arbre (41).

14. Une aiguille de biopsie (100) selon l'une quelconque des revendications précédentes, **caractérisée par** un clapet anti-retour (4) comportant un arbre (41) possédant au moins un réservoir (411) se trouvant sur l'arbre (41) et dans laquelle on collecte les échantillons prises, au moins une tête d'arbre (412) s'étendant d'une extrémité depuis l'arbre (41) et par laquelle passe une ligne de transfert (33), au moins un protrusion (413) se trouvant sur la partie inférieure de l'arbre (41) et permettant à l'arbre (41) de s'appuyer sur la pièce mobile (2).

15. Une aiguille de biopsie (100) selon l'une quelconque des revendications précédentes, **caractérisée par** un clapet anti retour (4) comportant au moins un trou de raccordement (431) se trouvant sur la tête d'arbre et tenant l'élément de raccordement (43).
